(19) Europäisches Patentamt
European Patent Office
Office européen des brevets

(11) Publication number: **0 160 573**
**A2**

(12) # EUROPEAN PATENT APPLICATION

(21) Application number: 85303102.9

(22) Date of filing: 01.05.85

(51) Int. Cl.⁴: C 07 D 239/36
C 07 D 239/22, A 61 K 31/5-05

(30) Priority: 02.05.84 GB 8411291

(43) Date of publication of application:
06.11.85 Bulletin 85/45

(84) Designated Contracting States:
AT BE CH DE FR GB IT LI LU NL SE

(71) Applicant: NYEGAARD & CO. A/S
Nycoveien 2 Postboks 4220
Oslo 4(NO)

(72) Inventor: Undheim, Kjell
17 Sverrestien
N-1310 Blommenholm Oslo(NO)

(72) Inventor: Benneche, Tore
2858-1 Coho Street
Madison Wisconsin 53713(US)

(74) Representative: Cockbain, Julian et al,
Frank B. Dehn & Co. Imperial House 15-19, Kingsway
London WC2B 6UZ(GB)

(54) **Pyrimidinone derivatives.**

(57) Bisulphite adducts of compounds of formula I

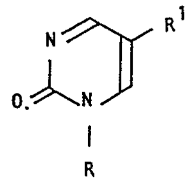

(I)

(wherein R represents a group $R^2-\overset{|}{C}H-X-(CR^4R^5)_nR^3$;

$R^1$ represents a halogen atom or a trifluoromethyl group;

$R^2$ represents a hydrogen atom or a $C_{1-4}$ alkyl, $C_{1-4}$ alkanoyl or phenyl group;

X represents an oxygen or a sulphur atom or a group $>NR^6$, where $R^6$ represents a formyl, $C_{1-4}$ alkanoyl or $C_{1-4}$ alkoxycarbonyl group;

$R^3$ represents a $C_{6-10}$ carbocyclic aromatic group or a heterocyclic group containing a 5- or 6- membered unsaturated heterocyclic ring which ring contains one or more heteroatoms selected from O, N and S and optionally carries a fused carbocyclic ring, which carbocyclic or heterocyclic group may carry one or more substituents selected from halogen atoms, $C_{1-4}$ alkyl, $C_{1-4}$ alkanoyl, $C_{1-4}$ alkoxy, $C_{1-4}$ alkoxycarbonyl, $C_{1-4}$ alkylthio, hydroxyl, nitro, cyano and formyl groups, $-NR^7R^8$ groups (where $R^7$ is a hydrogen atom or a $C_{1-4}$ alkyl group, and $R^8$ is a hydrogen atom or a $C_{1-4}$ alkyl, $C_{1-4}$ alkanoyl or aroyl group) and $C_{1-4}$ alkyl groups substituted by one or more hydroxy or $-NR^7R^8$ groups and halogen atoms;

n is an integer having the value 0 or 1; and

$R^4$ and $R^5$, which may be the same or different, each is a hydrogen atom or a $C_{1-6}$ alkyl group) and salts of such adducts have metaphase arrest abilities and desirably good water solubilities and may be useful in combatting abnormal cell proliferation.

EP 0 160 573 A2

PYRIMIDINONE DERIVATIVES

This invention relates to pyrimidinone derivatives, to processes for their preparation, and to pharmaceutical compositions containing them.

European Patent Applications Publication Nos. 56319 and 87326 disclose 5-halo-pyrimidin-2-ones which possess a metaphase arresting ability. This ability is useful for combating abnormal cell proliferation, which can be found in auto-immune diseases, and diseases such as cancers, leukaemias or cutaneous cellular proliferation, e.g. contact dermatitis or psoriasis.

We have found a new group of pyrimidin-2-one derivatives which have very good metaphase arresting ability and which are useful in combating abnormal cell proliferation.

In general, these compounds possess good water-solubility and by virtue of this are particularly easy to administer.

In particular, we have found that 4- and/or 6-unsubstituted 5-halo-pyrimidin-2-ones according to the European Patent Applications referred to above are capable of forming bisulphite adducts having metaphase arresting abilities and enhanced water solubilities.

According to one preferred aspect of the present invention we provide compounds being bisulphite adducts of compounds of formula I

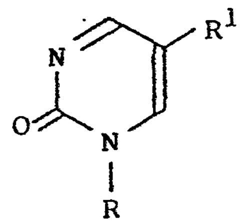

(I)

(wherein R represents a group $R^2-\overset{|}{C}H-X-(CR^4R^5)_nR^3$;

$R^1$ represents a halogen atom or a trifluoromethyl group;

$R^2$ represents a hydrogen atom or a $C_{1-4}$ alkyl, $C_{1-4}$ alkanoyl or phenyl group;

X represents an oxygen or a sulphur atom or a group $>NR^6$, where $R^6$ represents a formyl, $C_{1-4}$ alkanoyl or $C_{1-4}$ alkoxycarbonyl group;

$R^3$ represents a $C_{6-10}$ carbocyclic aromatic group or a heterocyclic group containing a 5- or 6- membered unsaturated heterocyclic ring which ring contains one or more heteroatoms selected from O, N and S and optionally carries a fused carbocyclic ring, which carbocyclic or heterocyclic group may carry one or more substituents selected from halogen atoms, $C_{1-4}$ alkyl, $C_{1-4}$ alkanoyl, $C_{1-4}$ alkoxy, $C_{1-4}$ alkoxycarbonyl, $C_{1-4}$ alkylthio, hydroxyl, nitro, cyano and formyl groups, $-NR^7R^8$ groups (where $R^7$ is a hydrogen atom or a $C_{1-4}$ alkyl group, and $R^8$ is a hydrogen atom or a $C_{1-4}$ alkyl, $C_{1-4}$ alkanoyl or aroyl group) and $C_{1-4}$ alkyl groups substituted by one or more hydroxy or $-NR^7R^8$ groups and halogen atoms;

n is an integer having the value 0 or 1; and

$R^4$ and $R^5$, which may be the same or different, each is a hydrogen atom or a $C_{1-6}$ alkyl group) and salts of such adducts.

The adducts according to the invention are sulphonates and are thus ionic materials capable of salt formation. Such salts are conveniently physiologically acceptable, water soluble salts and may advantageously be sodium, potassium, calcium, magnesium, ammonium or trialkylammonium salts.

The adducts of the invention are conveniently formed directly through an addition reaction, however the term "adduct" is used herein to refer to the final product irrespective of its manner of production and thus includes within its scope compounds produced other than by direct bisulphite addition to compounds of formula I.

Thus in a further aspect of the invention we provide compounds of formula II

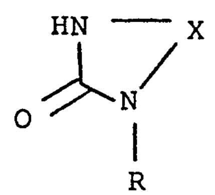

(II)

(wherein X represents the group

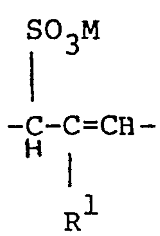

;

M represents a cation, preferably selected from sodium, potassium, calcium, magnesium, ammonium and trialkylammonium; and R and $R^1$ are as defined above) and salts thereof, separately or in admixture.

The compounds of formula II may be substituted at the 4- or 6-position having the formula IIa or IIb

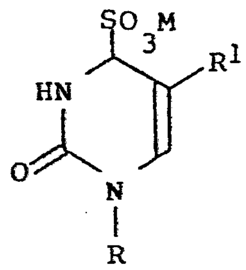

(IIa)

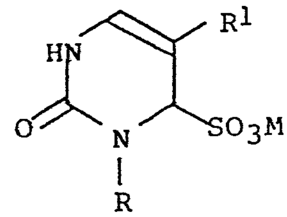

(IIb)

In the above definitions of the compounds according to the invention, the term "halogen" is used to mean fluorine, chlorine, bromine or iodine.

The group $R^2$ may be for example a methyl, ethyl, acetyl or phenyl group, but advantageously represents a hydrogen atom.

X may for example represent a group $>NR^6$ in which $R^6$ is a formyl, acetyl or ethoxycarbonyl group, but X advantageously represents an oxygen or sulphur atom.

Examples of the $C_{6-10}$ carbocyclic aromatic groups within the definition of the group $R^3$ are phenyl or naphthyl groups which may be substituted by the groups indicated above. Examples of the optional substituents which may be present on a phenyl or naphthyl group include one, two or more chlorine or fluorine atoms, or methyl, acetyl, methoxy, methoxycarbonyl, methylthio, hydroxyl, nitro, cyano, formyl, $-NH_2$, or hydroxymethyl groups. Substituents on the phenyl group may be present, for example, in the 2-, 3- and/or 4- positions.

$R^3$ may alternatively be a five or six-membered unsaturated heterocyclic ring which may contain up to four, preferably 1, 2 or 3, hetero atoms. Such heterocyclic rings include pyrimidinyl and imidazolyl rings, e.g. the pyrimidin-2-yl and

imidazol-2-yl rings. The five- or six-membered unsaturated heterocyclic ring may have another ring, fused to it, which may be a carbocyclic ring such as phenyl. Examples of the substituents which optionally may be present on the heterocyclic ring, or on any fused ring, are halogen atoms such as chlorine atoms or $C_{1-4}$ alkyl groups e.g. methyl groups.

$R^4$ and $R^5$ may each represent a hydrogen atom or a methyl group, but preferably each represents a hydrogen atom.

Examples of the group R include $R^2-\overset{|}{C}H-X-R^3$, and $-CH_2XCH_2R^3$, where $R^2$ is a hydrogen atom or a phenyl group, X is an oxygen or sulphur atom and $R^3$ is a phenyl group (optionally substituted by one or two chlorine atoms, or a methyl, acetyl, methoxy, methoxycarbonyl, nitro, cyano or formyl group) or a naphthyl group; and $-CH_2NR^6R^3$, where $R^6$ is an ethoxycarbonyl group and $R^3$ is a phenyl group.

Compounds of formula IIa are particularly interesting.

When the cation M in formulae IIa or IIb is a trialkylammonium ion the alkyl groups conveniently contain up to 4 carbon atoms and such a group may be for example a triethylammonium cation. Preferably, however, M is a sodium ion.

Preferred compounds of the present invention include the sodium, potassium, calcium, magnesium, ammonium or trialkylammonium salts of the bisulphite adducts of the compounds of formula I in which $R^1$ represents a chlorine atom and R represents the group

$$-\overset{R^2}{\overset{|}{C}}H-X-R^3, \quad -CH_2OCH_2R^3 \text{ or } -CH_2\overset{R^6}{\overset{|}{N}}R^3,$$

wherein $R^2$ is a hydrogen atom or a phenyl group, X is an oxygen or sulphur atom and $R^3$ is a phenyl group (optionally substituted by one or two chlorine atoms, or a methyl, acetyl, methoxy, methoxycarbonyl, nitro, cyano or formyl group) or a naphthyl group; or the group $-CH_2NR^6R^3$, where $R^6$ is an ethoxycarbonyl group and $R^3$ is a phenyl group.

Particularly interesting compounds include those of formula IIa and IIb in which:

M is a sodium, potassium, calcium, magnesium, ammonium or trialkylammonium ion, preferably a sodium, potassium, ammonium or trialkylammonium ion, especially a sodium ion;

$R^1$ is a chlorine atom: and

R is a group $R^2\text{-}\overset{|}{C}H\text{-}X(CH_2)_nR^3$, where $R^2$ is a hydrogen atom or a phenyl group, n is 0 or 1, X is an oxygen or sulphur atom and $R^3$ is a phenyl or naphthyl group, optionally substituted by one or two chlorine atoms or a methyl, acetyl, methoxy, methoxycarbonyl, nitro, cyano or formyl group; or n is O, X is the group $>NR^6$, where $R^6$ is an ethoxycarbonyl group, and $R^3$ is a phenyl group.

Particularly preferred compounds of this type are those of formula IIa or IIb, especially formula IIa, in which M is a sodium ion, $R^1$ is a chlorine atom and R is (a) a group $-CH_2OR^3$, where $R^3$ is a phenyl group substituted, preferably in the 4-position, by a formyl or acetyl group; (b) a group $-CH_2OCH_2R^3$, where $R^3$ is a phenyl group substituted, preferably in the 4-position, by a methoxy or methoxycarbonyl group; or (c) a group $-CH_2NR^6R^3$, where $R^6$ is ethoxycarbonyl and $R^3$ is phenyl.

Particularly interesting compounds according to the invention include the following:

sodium 5-chloro-1-(4-formylphenoxy)methyl-2-oxo-1,2,3,4-tetrahydropyrimidine-4-sulphonate;

sodium 5-chloro-1-(4-methoxybenzyloxy)methyl-2-oxo-1,2,3,4-tetrahydropyrimidine-4-sulphonate; sodium 1-(4-acetylphenoxy)methyl-5-chloro-2-oxo-1,2,3,4-tetrahydropyrimidine-4-sulphonate; sodium 5-chloro-1-[1-(3-trifluoromethylphenyloxy)ethyl]-2-oxo-1,2,3,4-tetrahydropyrimidine-4-sulphonate; sodium 5-chloro-1-(naphth-1-ylmethyloxy)methyl-2-oxo-1,2,3,4-tetrahydropyrimidine-4-sulfonate; and sodium 5-chloro-1- α-(phenyloxy)ethyl-2-oxo-1,2,3,4-tetrahydropyrimidine-4-sulfonate.

It will be appreciated that the compounds according to the invention, depending on the substituent groups present, may exist in optically active forms and all such forms, as well as mixtures thereof, including racemates, are included within the scope of the invention.

As indicated above, our tests have shown that the bisulphite adducts of the invention have a metaphase arresting ability. The test we have used to determine this ability is that using a growing culture of L1210 cells as described in European Patent Application Publication No. 87326.

The compounds according to the invention are thus of interest in combatting abnormal cell proliferation. Their use is based on the concept of using a drug to arrest the cell-division cycle reversibly in metaphase, so that subsequent treatment with a cytotoxic drug is less indiscriminate in its effects, as discussed in European Patent Application Publication No. 87326. As described therein, it will normally be necessary to have a knowledge of the cell cycle kinetics of both the normal and abnormal cells to be treated, and to employ this to achieve the optimum result in the use of the compounds of the invention.

The compounds according to the invention may be formulated for use in the conventional manner, and in a further aspect of the invention we provide

a pharmaceutical composition comprising an effective amount of an adduct or compound of formula II according to the invention (wherein the or any countercation is a physiologically acceptable cation) together with one or more pharmaceutical carriers or excipients.

The adducts according to the invention may be incorporated into pharmaceutical compositions of either solid or liquid forms. The compositions may, for example, be presented in forms suitable for administration by the rectal, parenteral or topical routes. Preferred forms include, for example suspensions, suppositories, creams, ointments, lotions and solutions e.g. for injection or infusion.

The active ingredient may be incorporated in excipients customarily employed in pharmaceutical compositions such as, for example, talc, gum arabic, lactose, starch, magnesium stearate, cocoa butter, aqueous or non-aqueous vehicles, fatty substances of animal or vegetable origin, paraffin derivatives, glycols, various wetting, dispersing or emulsifying agents and/or preservatives.

Advantageously, the compositions may be formulated as dosage units, each unit being adapted to supply a fixed dose of active ingredient. Suitable dosage units for adults contain from 50 mg to 1.0 g of active ingredient. The dosage, which may be varied according to the compound used, the subject treated and the complaint concerned, may, for example, be from 0.25 to 7.0 g in a day in adults.

In one aspect of the invention the compositions of the invention may take the form of compositions comprising an effective amount of a compound of formula I, or a physiologically acceptable addition salt thereof, formulated with an aqueous solution of a physiologically acceptable bisulphite (or metabisulphite) whereby the adduct is formed in situ in the composition. In such cases, it is convenient to use an excess of the physiologically

acceptable bisulphite (or metabisulphite) and the formulation of the compound of formula I with the bisulphite (or metabisulphite) solution is preferably carried out immediately or shortly before administration of the composition.

In another aspect, the invention provides the use of adducts or compounds of formula II according to the invention (wherein the or any countercation is physiologically acceptable) for the manufacture of a therapeutic agent for use in the treatment of the human or non-human animal body to effect metaphase arrest of cell growth.

In a further aspect, the invention provides the use of adducts or compounds of formula II according to the invention (wherein the or any countercation is physiologically acceptable) for the treatment of the human or non-human animal body to effect metaphase arrest of cell growth.

In a still further aspect, the invention provides a method of treatment of the human or non-human animal body to effect metaphase arrest of cell growth which method comprises administering to said body an effective amount of an adduct or compound of formula II according to the invention (wherein the or any countercation is physiologically acceptable).

In another aspect the invention provides a process for the preparation of a pharmaceutical composition comprising admixing an adduct or compound of formula II according to the invention (wherein the or any countercation is physiologically acceptable) with one or more pharmaceutical carriers or diluents.

The adducts of the invention may be prepared by bisulphite addition to compounds of formula I.

In one process, a bisulphite adduct of the invention may be prepared by addition of bisulphite to a pyrimidinone of formula I, where R and $R^1$ are as previously defined. The reaction may be

effected using a substance providing bisulphite ions, e.g. an appropriate metabisulphite, for example $M_2S_2O_5$ (where M is sodium, potassium, ammonium or trialkylammonium), $CaS_2O_5$ or $MgS_2O_2$, in an aqueous medium such as water or water with a co-solvent such as chloroform, dimethylformamide or dioxan optionally in the presence of a phase transfer catalyst such as benzyltrimethylammonium chloride. The reaction may be performed at for example 20° - 100°C, conveniently at 20° - 60°C.

The preparation of certain of the pyrimidinone starting materials for this process is described in European Patent Applications Publication Nos. 56319 and 87326. Other compounds of formula I whose preparation is not specifically disclosed in those publications may be prepared using analogous methods. Certain of these novel compounds, which possess particularly useful metaphase arrest activity and which fall within the scope of the general disclosure of European Patent Applications Publication Nos. 56319 and 87326 although not being specifically disclosed therein are particularly valuable as intermediates in the preparation of the adducts of the invention and so form another aspect of the present invention. Thus according to this further aspect, the present invention provides compounds of formula III

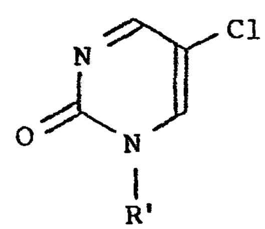

(III)

wherein R' represents the group $-CH_2OR'^3$ (in which $R'^3$ represents a phenyl group substituted in the 4-position by a formyl group) or $-CH_2OCH_2R'^3$ in which $R'^3$ represents a phenyl group substituted in the 3- or 4- position by a methyl group or in

the 3- and 4- positions by a chlorine atom) and salts thereof, being compounds useful in the preparation of the adducts or compounds of formula II according to the invention.

A particularly interesting compound of formula III is 5-chloro-1-(4-formylphenoxy)methyl-2(1*H*)-pyrimidinone.

The metaphase arresting ability of the compounds of formula III may be determined using the test described above and the compounds of formula III may be formulated for use as previously described for the bisulphite adducts of the invention and in another aspect, the invention accordingly provides pharmaceutical compositions containing at least one compound of formula III together with one or more pharmaceutical carriers or excipients.

The following non-limiting Examples are provided to illustate the invention. All temperatures are in °C.

Intermediate 1

4-(Methylthiomethoxy)benzaldehyde

Sodium hydride dispersion in liquid paraffin (80%; 2.64g) and sodium iodide (12.0g) were added successively to a solution of 4-hydroxybenzaldehyde (9.76g) in 1,2-dimethoxyethane (80 ml) at 4°C and the mixture stirred for 15 min before chloromethyl methyl sulfide (97%; 6.77 ml) was added. The resultant mixture was allowed to reach ambient temperature and was stirred for 6h, the volume reduced to ca. 1/3 at reduced pressure, the residue poured onto ice-water, the mixture extracted with ether, the washed and dried ($MgSO_4$) ether solution evaporated and the residue either purified by passage through silica gel column using chloroform : petroleum ether (1 : 1), or by a quick distillation; b.p. 96 -106°C/0.1 mmHg; $^1H$ NMR ($CDCl_3$): $\delta$ 2.28 ($CH_3S$), 5.23 ($SCH_2O$), 7.0 - 8.0 (Ph), 9.93 (CHO).

Intermediates 2 to 5 were prepared by a similar procedure:

Intermediate 2

3-(Methylthiomethoxy)benzonitrile (7.9g) from sodium hydride (1.50 g), sodium iodide (6.75 g), 3-hydroxybenzonitrile (5.40 g) and chloromethyl sulfide (3.8 ml); $^1H$ NMR ($CDCl_3$): $\delta$ 2.93 ($CH_3S$), 5.18 ($SCH_2O$), 7.2 - 7.5 (Ar).

Intermediate 3

3-(Methylthiomethoxy)methyltoluene from 3-methylbenzyl alcohol b.p. 76 - 78°C/1.0 mmHg. $^1H$ NMR ($CDCl_3$): $\delta$ 2.12 ($CH_3$), 2.33 ($CH_3$), 4.48 ($CH_2$), 4.57 ($CH_2$), 7.1 - 7.3 (Ar).

Intermediate 4

4-(Methylthiomethoxy)methyltoluene from 4-methylbenzyl alcohol b.p. 60°C/0.05 mmHg. $^1H$ NMR ($CDCl_3$): δ 2.18 ($CH_3$), 2.33 ($CH_3$), 4.53 ($CH_2$), 4.64 ($CH_2$), 7.2 (Ar).

Intermediate 5

1-(Methylthiomethoxy)methyl-3,4-dichlorobenzene from 3,4-dichlorobenzyl alcohol. The crude product was used directly in the subsequent reaction.

Intermediate 6

4-(Chloromethoxy)benzaldehyde
Sulfuryl chloride (3.2 ml), in dichloromethane (40 ml) was added dropwise during 10 min at 4°C to a solution of Intermediate 1 (7.4 g) in dichloromethane (60 ml). The mixture was stirred at ambient temperature for 30 min, the solvent and the methanesulfenyl chloride (which is formed in the reaction) evaporated off at reduced pressure and the residue purified by distillation to yield the title compound (4.2 g), b.p. 86 - 90°C/0.1 mmHg. $^1H$ NMR ($CDCl_3$): δ 5.96 ($CH_2Cl$), 7.0 - 8.0 (Ar), 9.95 (CHO).

Intermediates 7 to 10 were prepared by a similar procedure:

Intermediate 7

3-(Chloromethoxy)benzonitrile from Intermediate 2; b.p. 80 - 82°C/0.1 mmHg. $^1H$ NMR ($CDCl_3$): δ 5.90 ($CH_2Cl$), 7.2 - 7.4 (Ar).

Intermediate 8

3-(Chloromethoxy)methyltoluene from Intermediate 3. $^1H$ NMR ($CCl_4$): δ 2.35 ($CH_3$), 4.62 ($SCH_2O$), 5.38 ($OCH_2Cl$), 7.0 - 7.2 (Ar).
The crude product was used in the subsequent reaction without any further purification.

Intermediate 9

4-(Chloromethoxy)methyltoluene from Intermediate 4. $^1H$ NMR ($CDCl_3$): δ 2.34 ($CH_3$), 4.70 ($PhCH_2$), 5.48 ($CH_2Cl$), 7.23 (Ar).
The crude product was used in the subsequent reaction without any further purification.

Intermediate 10

1-(Chloromethoxy)methyl-3,4-dichlorobenzene from Intermediate 5. $^1H$ NMR ($CDCl_3$): δ 4.71 ($PhCH_2$), 5.55 ($CH_2Cl$), 7.1 - 7.6 (Ar).
The crude product was used in the subsequent reaction without any further purification.

The following Intermediates were prepared using a similar procedure to that used for the preparation of the compound of Example 5(a):

Intermediate 11

5-Chloro-1-(3-cyanophenoxy)methyl-2(1H)-pyrimidinone (3.3 g) from Intermediate 7 (4.90 g), 5-chloro-2(1H)-pyrimidinone (3.90 g) and triethylamine (4.0 ml) in dichloromethane (90 ml); m.p. 260°C; $^1H$ NMR (DMSO-$d_6$/$CDCl_3$): δ 5.82 ($CH_2$), 7.3 - 7.5 (Ar), 8.60 (H-4, H-6).

Intermediate 12

5-Chloro-1-(4-nitrophenoxy)methyl-2(1H)-pyrimidinone (3.3 g) from 1-chloromethoxy-4-nitrobenzene (4.4g), 5-chloro-(1H)-pyrimidinone (3.1 g) and triethylamine (3.2 ml) in dichloromethane (75 ml); m.p. 260°C. $^1$H NMR (TFA): δ 6.33 ($CH_2$), 7.0 - 7.3 and 8.2 - 8.5 (Ar), 9.2 - 9.3 (H-4, H-6).

Example 1

(a) 5-Chloro-1-(4-formylphenoxy)methyl-2(1H)-pyrimidinone

Intermediate 6 (14.9 g) in dichloromethane (100 ml) was added dropwise with stirring during 15 min at 4°C to a solution prepared from 5-chloro-2(1H)-pyrimidinone hydrochloride (11.4 g) and triethylamine (12.2 ml) in dichloromethane (300 ml). The resultant mixture was stirred at ambient temperature for 18 h when the precipitate was collected by filtration, the solid washed with water and triturated with acetone and ether to furnish the title compound. A second crop was obtained by the addition of chloroform (100 ml) to the filtrate which was washed with water, the dried ($MgSO_4$) solution evaporated and the residue triturated with ether. The total yield of title compound was 14.0 g. m.p. 182°C. $^1$H NMR ($CDCl_3$): δ 6.00 ($CH_2$), 7.8 - 8.0 (H-6, Ar), 8.67 (H-4), 10.00 (CHO).

The following compounds were prepared by a similar procedure:

(b) 5-Chloro-1-(3-methylbenzyloxy)methyl-2(1H)-pyrimidinone (4.0 g) from Intermediate 8 (6.83 g), 5-chloro-2(1H)-pyrimidinone (5.22 g) and triethylamine (5.60 ml) in dichloromethane (90 ml); m.p. 108°C. $^1$H NMR ($CDCl_3$): δ 2.33 ($CH_3$), 4.62 ($OCH_2Ph$), 5.33

($NCH_2O$), 7.0 - 7.3 (Ar), 7.78 (H-6, J 3 Hz), 8.48 (H-4).

(c) 5-Chloro-1-(4-methylbenzyloxy)methyl-2(1H)-pyrimidinone (2.62 g) from Intermediate 9 (2.90 g), 5-chloro-2(1H)-pyrimidinone (2.22 g) and triethylamine (1.72 g) in dichloromethane (130 ml); m.p. 150°C. $^1H$ NMR (DMSO-$d_6$): δ 2.27 ($CH_3$), 4.58 ($OCH_2Ar$), 5.29 ($NCH_2O$), 7.17 (Ar), 8.41 (H-6, J 4 Hz), 8.60 (H-4, J 4 Hz).

(d) 5-Chloro-1-(3,4-dichlorobenzyloxy)methyl-2(1H)-pyrimidinone (4.2 g) from Intermediate 10 (5.64 g), 5-chloro-2(1H)-pyrimidinone (3.26 g) and triethylamine (2.53 g) in dichloromethane (150 ml); m.p. 225°C.

(e) 5-Chloro-1-(1-naphthyloxy)methyl-2(1H)-pyrimidinone

In dichloromethane and in the presence of triethylamine 5-chloro-2(1H)-pyrimidinone was reacted with a chloromethoxy reagent to produce the title compound. The chloromethoxy reagent was prepared by reacting chloromethyl methyl sulfide with the sodium salt of α-naphthol in the presence of sodium iodide and by reacting the resultant product with sulfuryl chloride. $^1H$ NMR ($CDCl_3$): δ 5.95 ($CH_2O$), 8.0 (H-6), 8.65 (H-4), 7.0-8.4 (Ar).

(f) 5-Chloro-1-[1-(4-chlorophenyloxy)ethyl]-2(1H)-pyrimidinone

5-Chloro-2(1H)-pyrimidinone was reacted with α-chloroethyloxy-4-chlorobenzene in dichloromethane and in the presence of triethylamine to produce the title compound. $^1H$ NMR ($CCl_4$/acetone-$d_6$): δ 1.95 ($C\underline{H}_3CHO$), 6.6 ($CH_3C\underline{H}O$), 7.95 (H-6), 8.40 (H-4), 7.0-7.5 (Ar).

(g) 5-Chloro-1-(naphth-1-ylmethyloxy)methyl-2(1H)-pyrimidinone

5-Chloro-2(1H)-pyrimidinone was reacted with 1-chloromethyloxymethylnaphthalene in dichloromethane and in the presence of triethylamine to produce the title compound. $^1$H NMR: δ 5.05 ($CH_2O$), 5.25 ($OCH_2$), 7.1-8.0 (Ar, H-6), 8.20 (H-4).

(h) 5-Chloro-1-(1-benzyloxyethyl)-2(1H)-pyrimidinone

5-Chloro-2(1H)-pyrimidinone was reacted with α-chloroethyloxy-methyl-benzene in dichloromethane and in the presence of triethylamine to produce the title compound. $^1$H NMR ($CDCl_3$): δ 1.60 ($C\underline{H}_3CHO$), 4.60 ($CH_2O$), 6.05 ($CH_3C\underline{H}O$), 7.3 (Ar), 7.9 (H-6), 8.55 (H-4).

(i) 5-Iodo-1-(3-methoxybenzyloxy)methyl-2(1H)-pyrimidinone

5-Iodo-2(1H)-pyrimidinone was reacted with 3-chloromethyloxymethylanisole in dichloromethane and in the presence of triethylamine to produce the title compound. $^1$H NMR ($CDCl_3$): δ 3.80 ($CH_3O$), 4.70 ($CH_2O$), 5.35 ($CH_2O$), 6.8-7.3 (Ar), 8.45 (H-6), 8.65 (H-4).

(j) 5-Chloro-1-[1-(4-methyloxycarbonylphenylsulfenyl)-ethyl]-2(1H)-pyrimidinone

5-Chloro-2(1H)-pyrimidinone was reacted with methyl 4-chloroethylsulfenylbenzoate in dichloromethane and in the presence of triethylamine to produce the title compound. $^1$H NMR (DMSO-$d_6$): δ 1.80 ($C\underline{H}_3CHS$), 6.40 ($CH_3C\underline{H}S$), 7.36-8.08 (Ar), 8.60 (H-6), 8.77 (H-4).

(k) 1-(4-Acetylphenyloxy)methyl-5-trifluoromethyl-2(1H)-pyrimidinone

5-Trifluoromethyl-2(1H)-pyrimidinone was reacted with 4-chloromethyloxyacetophenone in dichloromethane and in the presence of triethylamine to

produce the title compound. $^{1}H$ NMR ($CDCl_3$): δ 2.56 ($CH_3$), 6.20 ($CH_2O$), 7.1-8.0 (Ar), 8.36 (H-6), 8.81 (H-4).

(l) 1-(4-Formylphenyloxy)methyl-5-trifluoromethyl-2(1H)-pyrimidinone

5-Trifluoromethyl-2(1H)-pyrimidinone was reacted with 4-chloromethyloxybenzaldehyde in dichloromethane and in the presence of triethylamine to produce the title compound. $^{1}H$ NMR ($CDCl_3$): δ 6.06 ($CH_2O$), 7.2-7.9 (Ar), 8.33 (H-6), 8.80 (H-4), 9.93 (CHO).

Example 2

(a) Sodium 5-chloro-1-(4-formylphenoxy)methyl-2-oxo-1,2,3,4-tetrahydropyrimidine-4-sulfonate and Sodium 5-chloro-1-(4-formylphenoxy)methyl-2-oxo-1,2,3,6-tetrahydropyrimidine-6-sulphonate

37% aq. $NaHSO_3$ (2.02 ml) was added to a suspension of the compound of Example 1(a) (2.08 g) in water (40 ml). The pH was adjusted to ca. 7, and the mixture was stirred at ambient temperature for 20 h. The almost water clear solution was filtered and freeze-dried to furnished the mixture of the title 4- and 6- isomers in the ratio 3:1.

4-Isomer: $^{1}H$ NMR (DMSO-$d_6$): δ 4.36 (H-4, s), 5.28 and 5.57 ($CH_2O$, J 9 Hz), 6.87 (H-6,s), 7.1- 8.0 (Ar), 9.88 (CHO).

6-Isomer: $^{1}H$ NMR (DMSO-$d_6$): δ 4.63 (H-6, s), 5.48 and 5.98 ($CH_2O$, J 9 Hz), 6.38 (H-4, s), 7.1 - 8.0 (Ar), 9.88 (CHO).

The following compounds were prepared by a similar procedure:

(b) Sodium 5-chloro-1-(4-acetylphenoxy)methyl-2-oxo-1,2,3,4-tetradihydropyrimidine-4-sulfonate and Sodium 5-chloro-1-(4-acetylphenoxy)methyl-2 oxo-1,2,3,6-tetrahydropyrimidine-6-sulphonate from 5-chloro-1-(4-acetylphenoxy)methyl-2 (1H) pyrimidinone and aq. $NaHSO_3$. The 4- and 6-isomers were obtained in the ratio 3 : 1.

4-Isomer: $^1H$ NMR ($D_2O$): $\delta$ 2.53 (MeCO), 4.85 (H-4, s), 5.27 and 5.62 ($CH_2O$, *J* 9 Hz), 6.78 (H-6,s), 6.9 -8.0 (Ar).

6-Isomer: $^1H$ NMR ($D_2O$): $\delta$ 2.53 (MeCO), 5.18 (H-6, s), ca. 5.4 and 5.9 ($CH_2O$, *J* ca. 13 Hz), 6.55 (H-4, s), 6.9 - 8.0 (Ar).

(c) Sodium 1-(4-acetylphenyloxy)methyl-2-oxo-5-trifluoromethyl-1,2,3,4-tetrahydropyrimidine-4-sulfonate and sodium 1-(4-acetylphenyloxy)methyl-2-oxo-5-trifluoromethyl-1,2,3,6-tetrahydropyrimidine-4-sulfonate from 1-(4-acetylphenyloxy)methyl-5-trifluoromethyl-2(1H)-pyrimidinone and aq. Na $HSO_3$. After freeze-drying the 4- and 6-isomers were obtained in the ratio 3:2.

4-Isomer: $^1H$ NMR ($D_2O$): $\delta$ 2.55 ($CH_3$), 5.02 (H-4), 5.26 and 5.44 ($CH_2O$), 6.9-8.0 (Ar), 7.35 (H-6).

6-Isomer: $^1H$ NMR ($D_2O$): $\delta$ 2.55 ($CH_3$), 5.35 (H-6), 5.97 and 6.23 ($CH_2O$), 6.9-8.0 (Ar), 7.10 (H-4).

If instead of freeze-drying, the aqueous solution is concentrated and left at ambient temperature the 4-isomer is selectively precipitated.

(d) Sodium 1-(4-formylphenyloxy)methyl-2-oxo-5-trifluoromethyl-1,2,3,4-tetrahydropyrimidine-4-sulfonate and sodium 1-(4-formylphenyloxy)methyl-2-oxo-5-trifluoromethyl-1,2,3,6-tetrahydropyrimidine-4-sulfonate from 1-(4-formylphenyloxy)methyl-5-trifluoromethyl-2(1H)-pyrimidinone and aq. Na $HSO_3$. After freeze-drying the 4- and 6-isomers were obtained in the ratio 3:1.

4-Isomer: $^1H$ NMR ($D_2O$): δ 5.07 (H-4), 5.35 and 5.57 ($CH_2O$), 7.0-8.1 (Ar), 7.43 (H-6), 9.73 (CHO).
6-Isomer: $^1H$ NMR ($D_2O$): δ 5.47 (H-6), 6.00 and 6.18 ($CH_2O$), 7.0-8.1 (Ar), 7.13 (H-4), 9.73 (CHO).

If instead of freeze-drying, the aqueous solution is concentrated to a small volume the 4-isomer is selectively precipitated.

Example 3

(a) Sodium 5-chloro-1-(4-formylphenoxy)methyl-2-oxo-1,2,3,4-tetrahydropyrimidine-4-sulfonate

37% aq. $NaHSO_3$ (5.4 ml) was added to a suspension of a compound of Example 1(a) (4.5 g) in water (40 ml). The pH was adjusted to ca. 7 and the mixture stirred at ambient temperature for 4h. Thereafter the mixture was heated at 100°C for 5 - 10 min, the almost clear solution filtered hot and the filtrate left to stand in the cold (5°C). The crystalline precipitate obtained (4.8 g) was recrystallized from water (20 ml) to furnished the title compound (3.1 g). $^1H$ NMR (DMSO-$d_6$): δ 4.36 (H-4, s), 5.28 and 5.57 ($CH_2O$, J 9 Hz), 6.87 (H-6, s), 7.1 - 8.0 (Ar), 9.88 (CHO).

The following compounds were prepared by a similar procedure:

(b) Sodium 1-(4-acetylphenoxy)methyl-5-chloro-2-oxo-1,2,3,4-tetrahydropyrimidine-4-sulfonate from 1-(4-acetylphenoxy)methyl-5-chloro-2(1H)-pyrimidinone and aq. $NaHSO_3$. $^1H$ NMR ($D_2O$): δ 2.53 ($CH_3CO$), 4.85 (H-4, s), 5.27 and 5.62 ($CH_2O$, J 9 Hz), 6.78 (H-6 s), 6.9 - 8.0 (Ar).

(c) Sodium 5-chloro-1-(3-cyanophenoxy)methyl-2-oxo-1,2,3,4-tetrahydropyrimidine-4-sulfonate from

Intermediate 11 and aq. $NaHSO_3$; $^1H$ NMR (DMSO-$\underline{d}_6$): δ 4.03, (H-4,d, $\underline{J}$ 3 Hz), 5.27 and 5.52 ($CH_2O$, $\underline{J}$ 9 Hz), 6.85 (H-6, s), 7.3 - 7.8 (Ar).

(d) Sodium 5-chloro-1-(4-nitrophenoxy)methyl-2-oxo-1,2,3,4-tetrahydropyrimidine-4-sulfonate from Intermediate 12 and aq. $NaHSO_3$; $^1H$ NMR (DMSO-$\underline{d}_6$): δ 4.27 (H-4, s), 5.27 and 5.53 ($CH_2$, $\underline{J}$ 9 Hz), 6.83 (H-6, s), 7.1 -7.3 and 8.0 - 8.3 (Ar), 7.6 - 7.7 (NH).

(e) Sodium 5-chloro-1-(2-naphthoxy)methyl-2-oxo-1,2,3,4-tetrahydropyrimidine-4-sulfonate from 5-chloro-1-(2-naphthoxy)methyl-2(1$\underline{H}$)-pyrimidinone and aq. $NaHSO_3$; $^1H$ NMR (DMSO-$\underline{d}_6$): 4.38 (H-4, d, $\underline{J}$ 2 Hz), 5.30 and 5.60 ($CH_2O$, $\underline{J}$ 9 Hz), 6.88 (H-6, s), 7.1 - 8.0 (Ar).

(f) Sodium 5-chloro-1-α-(4-chlorophenylsulfenyl)benzyl-2-oxo-1,2,3,4-tetrahydropyrimidine-4-sulfonate from 5-chloro-1-α-(4-chlorophenylsulfenyl)-2(1$\underline{H}$)-pyrimidinone and aq. $NaHSO_3$; $^1H$ NMR (DMSO-$\underline{d}_6$): δ 4.31 (H-4, d, $\underline{J}$ 3 Hz), 6.8 (H-6, s), 7.2- 7.8 (CH, Ar).

(g) Sodium 5-chloro-1-(N-ethoxycarbonylanilino)methyl-2-oxo-1,2,3,4-tetrahydropyrimidine-4-sulfonate from 5-chloro-1-(N-ethoxycarbonylanilino)methyl-2(1$\underline{H}$)-pyrimidinone and aq. $NaHSO_3$;$^1H$ NMR (DMSO-$\underline{d}_6$): δ 1.10 and 4.10 ($C_2H_5$), 4.35 (H-4, s), 5.1 - 5.5 ($NCH_2N$), 6.67 (H-6, s), 7.3 (Ar).

(h) Sodium 5-chloro-1-(3-methylbenzyloxy)methyl-2-oxo-1,2,3,4-tetrahydropyrimidine-4-sulfonate from the compound of Example 1(b) and aq. $NaHSO_3$; $^1H$ NMR (DMSO-$\underline{d}_6$): δ 2.28 ($CH_3$), 4.30 (H-4, d,

*J* 3 Hz), 4.45 ($OCH_2Ar$, s), 4.75 and 4.97 ($NCH_2O$, *J* 10 Hz), 6.67 (H-6, s), 7.0 - 7.3 (Ar), 7.45 (NH, d, *J* 3 Hz).

(i) Sodium 5-chloro-1-(4-methylbenzyloxy)methyl-2-oxo-1,2,3,4-tetrahydropyrimidine-4-sulfonate from compound of Example 1(c) and aq. $NaHSO_3$; $^1H$ NMR (DMSO-$d_6$): δ 2.30 ($CH_3$), 4.37 (H-4, s), 4.43 and 4.47 ($OCH_2Ar$), 4.73 and 4.97 ($NCH_2O$, *J* 10 Hz), 6.69 (H-6, s), 7.20 (Ar).

(j) Sodium 5-chloro-1-(4-methoxybenzyloxy)methyl-2-oxo-1,2,3,4-tetrahydropyrimidine-4-sulfonate from 5-chloro-1-(4-methoxybenzyloxy)methyl-2(1H)-pyrimidinone and aq. $NaHSO_3$.

(k) Sodium 5-chloro-1-(4-methyloxycarbonylbenzyloxy)-methyl-2-oxo-1,2,3,4-tetrahydropyrimidine-4-sulfonate from 5-chloro-1-(4-methyloxycarbonylbenzyloxy)-methyl-2(1H)-pyrimidinone and aq. $NaHSO_3$; $^1H$ NMR (DMSO-$d_6$): δ 3.87 ($OCH_3$), 4.33 (H-4, s), 4.55 and 4.60 ($OCH_2Ar$), 7.76 and 5.03 ($NCH_2O$, *J* 10 Hz), 6.73 (H-6, s), 7.3 - 8.1 (Ar).

(l) Sodium 5-chloro-1-(3,4-dichlorobenzyloxy)methyl-2-oxo-1,2,3,4-tetrahydropyrimidine-2-sulfonate from the compound of Example 1(d) and aq. $NaHSO_3$; $^1H$ NMR (DMSO-$d_6$): δ 4.33 and 4.51 ($OCH_2Ar$, *J* 3 Hz), 4.5 (H-4, s), 4.77 and 5.07 ($NCH_2O$, *J* 11 Hz), 6.77 (H-6), 7.2 - 7.8 (Ar).

(m) Sodium 5-chloro-1-(1-naphthyloxy)methyl-2-oxo-1,2,3,4-tetrahydropyrimidine-4-sulfonate from 5-chloro-1-(1-naphthyloxy)methyl-2(1H)-pyrimidinone and aq. $NaHSO_3$. $^1H$ NMR (DMSO-$d_6$): δ 4.3 (H-4), 5.3 and 5.6 ($CH_2O$), 6.9 (H-6).

(n) Sodium 5-chloro-1-[1-(3-trifluoromethylphenyloxy)-ethyl]-2-oxo-1,2,3,4-tetrahydropyrimidine-4-sulfonate from 5-chloro-1-[1-(3-trifluoromethylphenyloxy)ethyl]-2(1H)-pyrimidinone and aq. $NaHSO_3$. $^1H$ NMR (DMSO-$d_6$): δ 1.50 ($CH_3CHO$), 4.25 (H-4), 6.45 ($CH_3CHO$), 6.60 (H-6), 7.3-7.6 (Ar).

(o) Sodium 5-chloro-1-[1-(4-chlorophenyloxy)ethyl]-2-oxo-1,2,3,4-tetrahydropyrimidine-4-sulfonate from 5-chloro-1-[1-(4-chlorophenyloxy)ethyl]-2-(1H)-pyrimidinone and aq. $NaHSO_3$. $^1H$ NMR (DMSO-$d_6$/$D_2O$): δ 1.50 ($CH_3CHO$, d, J 6Hz), 4.37 (H-4), 6.42 ($CH_3CHO$, q, J 6Hz), 6.70 (H-6), 7.0-7.7( Ar).

(p) Sodium 5-chloro-1-[1-(4-methyloxycarbonylphenyl-sulfenyl)ethyl]-2-oxo-1,2,3,4-tetrahydropyrimidine-4-sulfonate from 5-chloro-1-[1-(4-methyloxycarbonylphenyl-sulfenyl)ethyl]-2(1H)-pyrimidinone and aq. $NaHSO_3$. $^1H$ NMR (DMSO-$d_6$): δ 1.50 ($CH_3CHS$), 3.85 ($OCH_3$), 4.2 (H-4), 6.25 ($CH_3CHS$), 6.80 (H-6), 7.4-8.0 (Ar).

(q) Sodium 5-chloro-1-(naphth-1-ylmethyloxy)methyl-2-oxo-1,2,3,4-tetrahydropyrimidine-4-sulfonate from 5-chloro-1-(naphth-1-ylmethyloxy)methyl-2-(1H)-pyrimidinone and aq. $NaHSO_3$. $^1H$ NMR (DMSO-$d_6$): δ 4.80-5.20 (H-4, $CH_2Ar$, $NCH_2$), 6.80 (H-6), 7.30-8.30 (Ar).

(r) Sodium 5-chloro-1-[N-(ethyloxycarbonyl)benzylamino]-methyl-2-oxo-1,2,3,4-tetrahydropyrimidine-4-sulfonate from 5-chloro-1-[N-(ethyloxycarbonyl)benzylamino]methyl-2(1H)-pyrimidinone and aq. $NaHSO_3$. $^1H$ NMR (DMSO-$d_6$): δ 1.21 ($CH_3$), 4.14 ($OCH_2$), 4.33 (H-4), 4.50 ($CH_2Ph$), 4.87 ($NCH_2N$), 6.31 (H-6), 7.32 (Ar).

(s) Sodium 5-chloro-1-(1-benzyloxyethyl)-2-oxo-1,2,3,4-tetrahydropyrimidine-4-sulfonate from 5-chloro-1-(1-benzyloxyethyl)-2(1H)-pyrimidinone

and aq. $NaHSO_3$. $^1H$ NMR (DMSO-$d_6$): δ 1.28 ($CH_3CHO$), 4.30 (H-4), 4.40 ($CH_2O$), 5.65 ($CH_3CHO$), 6.55 (H-6), 7.3 (Ar).

(t) Sodium 5-chloro-1-α-(benzylsulfenyl)benzyl-2-oxo-1,2,3,4-tetrahydropyrimidine-4-sulfonate from 5-chloro-1-α-(benzylsulfenyl)benzyl-2(1H)-pyrimidinone and aq. $NaHSO_3$. $^1H$ NMR (DMSO-$d_6$): δ 3.9 (H-4), 4.0 and 4.4 ($SCH_2$), 6.6 (CHS), 6.9 (H-6).

(u) Sodium 5-chloro-1-α-(phenyloxy)ethyl-2-oxo-1,2,3,4-tetrahydropyrimidine-4-sulfonate from 5-chloro-1-α-(phenyloxy)ethyl-2(1H)-pyrimidinone and aq. $NaHSO_3$.

(v) Sodium 5-iodo-1-(3-methoxybenzyloxy)methyl-2-oxo-1,2,3,4-tetrahydropyrimidine-4-sulfonate from 5-iodo-1-(3-methoxybenzyloxy)methyl-2(1H)-pyrimidinone and aq. $NaHSO_3$. $^1H$ NMR (DMSO-$d_6$): δ 3.8 (H-4), 4.3-5.0 ($CH_2OCH_2$, H-4), 6.8 (H-6), 6.9-7.3 (Ar).

Example 4

Sodium 5-chloro-1-(3-cyanophenoxy)methyl-2-oxo-1,2,3,4-tetrahydropyrimidine-4-sulfonate

A mixture of Intermediate 11 (0.52 g) and sodium metabisulfite (0.57 g) in DMF (15 ml) and water (2 ml) was stirred at 60° for 1h before the solvent was distilled off at reduced pressure. Water (10 ml) was added to the residue and the solid was collected, washed with acetone and ether to yield the title compound (0.5 g). NMR identical to that reported in Example 3(c).

Example 5

Sodium 5-chloro-1-(3-cyanophenoxy)methyl-2-oxo-1,2,3,4-tetrahydropyrimidine-4-sulfonate

A mixture of Intermediate 11 (0.52 g) sodium metabisulfite (0.76 g) and triethylbenzylammonium chloride (ca. 50 mg) in $CHCl_3$ (15 ml) and water (2 ml) was stirred at ambient temperature for 48 h. The solid material was collected, washed with water, hot chloroform, acetone and ether to yield the title compound (0.4 g). NMR identical to that reported in Example 3(c).

CLAIMS:

1. Compounds being bisulphite adducts of compounds of formula I

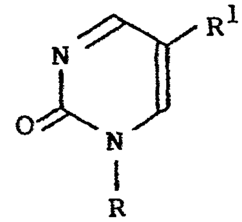

(I)

(wherein R represents a group $R^2$-CH-X-$(CR^4R^5)_nR^3$;

$R^1$ represents a halogen atom or a trifluoromethyl group;

$R^2$ represents a hydrogen atom or a $C_{1-4}$ alkyl, $C_{1-4}$ alkanoyl or phenyl group;

X represents an oxygen or a sulphur atom or a group >NR$^6$, where $R^6$ represents a formyl, $C_{1-4}$ alkanoyl or $C_{1-4}$ alkoxycarbonyl group;

$R^3$ represents a $C_{6-10}$ carbocyclic aromatic group or a heterocyclic group containing a 5- or 6- membered unsaturated heterocyclic ring which ring contains one or more heteroatoms selected from O, N and S and optionally carries a fused carbocyclic ring, which carbocyclic or heterocyclic group may carry one or more substituents selected from halogen atoms, $C_{1-4}$ alkyl, $C_{1-4}$ alkanoyl, $C_{1-4}$ alkoxy, $C_{1-4}$ alkoxycarbonyl, $C_{1-4}$ alkylthio, hydroxyl, nitro, cyano and formyl groups, $-NR^7R^8$ groups (where $R^7$ is a hydrogen atom or a $C_{1-4}$ alkyl group, and $R^8$ is a hydrogen atom or a $C_{1-4}$ alkyl, $C_{1-4}$ alkanoyl or aroyl group) and $C_{1-4}$ alkyl groups substituted by one or more hydroxy or $-NR^7R^8$ groups and halogen atoms;

n is an integer having the value 0 or 1; and

$R^4$ and $R^5$, which may be the same or different, each is a hydrogen atom or a $C_{1-6}$ alkyl group)

and salts of such adducts.

2. Compounds of formula II

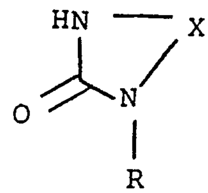

(II)

(wherein X represents the group

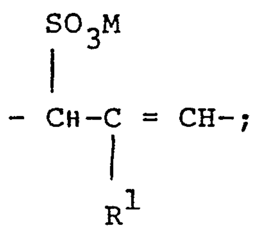

M represents a cation; and R and $R^1$ are as defined in claim 1).

3. Compounds as claimed in claim 2 of formula (IIa)

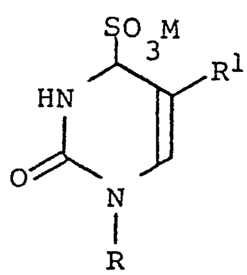

(IIa)

(wherein M, R and $R^1$ are as defined in claim 2).

4. Compounds as claimed in either of claims 2 and 3 wherein M represents a sodium, potassium, calcium, magnesium, ammonium or tri($C_{1-4}$alkyl)ammonium ion.

5. Compounds as claimed in any one of claims 1 to 4 wherein $R^2$ represents a hydrogen atom or a methyl, ethyl, acetyl or phenyl group, $R^3$ represents

an optionally substituted phenyl or naphthyl group or an optionally substituted 5 or 6 membered heterocyclic ring having up to four heteroatoms, n is 0 or 1, $R^4$ and $R^5$, which may be the same or different, each represents a methyl group or a hydrogen atom, and X represents an oxygen or sulphur atom or a group $>N-R^6$ in which $R^6$ is a formyl, acetyl or ethoxycarbonyl group.

6. Compounds as claimed in any one of the preceding claims wherein R represents a group of formula $R^2-CH-X-R^3$ or $-CH_2-X-CH_2-R^3$ (wherein $R^2$ represents a hydrogen atom or a phenyl group, X represents an oxygen or sulphur atom and $R^3$ represents a naphthyl group or a phenyl group optionally substituted by one or two substitutents selected from the group consisting of chlorine atoms and methyl, acetyl, methoxy, methoxycarbonyl, nitro, cyano and formyl groups) or a group of formula $-CH_2NR^6R^3$ (in which $R^6$ is an ethoxycarbonyl group and $R^3$ is a phenyl group).

7. Compounds of formula II as claimed in claim 2 wherein M represents a sodium ion, $R^1$ represents a chlorine atom and R represents a group $-CH_2OR^3$ (in which $R^3$ represents a phenyl group substituted by a formyl or acetyl group), a group $-CH_2OCH_2R^3$ (in which $R^3$ represents a phenyl group substituted by a methoxy or methoxycarbonyl group) or a group

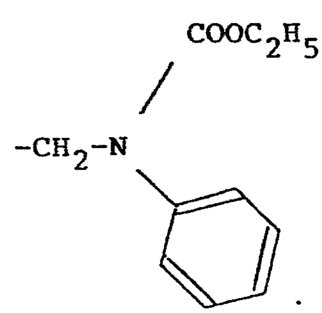

8. A compound as claimed in claim 1 being a compound selected from the group consisting of: sodium 5-chloro-1-(4-formylphenoxy)methyl-2-oxo-1,2,3,4-tetrahydropyrimidine-4-sulphonate; sodium 5-chloro-1-(4-methoxybenzyloxy)methyl-2-oxo-1,2,3,4-tetrahydropyrimidine-4-sulphonate; sodium 1-(4-acetylphenoxy)methyl-5-chloro-2-oxo-1,2,3,4-tetrahydropyrimidine-4-sulphonate; sodium 5-chloro-1-[1-(3-trifluoromethylphenyloxy)ethyl]-2-oxo-1,2,3,4-tetrahydropyrimidine-4-sulphonate; sodium 5-chloro-1-(naphth-1-ylmethyloxy)methyl-2-oxo-1,2,3,4-tetrahydropyrimidine-4-sulfonate; and sodium 5-chloro-1- α-(phenyloxy)ethyl-2-oxo-1,2,3,4-tetrahydropyrimidine-4-sulfonate.

9. A pharmaceutical composition comprising an effective amount of a compound as claimed in any one of the preceding claims (wherein the or any countercation is a physiologically acceptable cation) together with one or more pharmaceutical carriers or excipients.

10. A pharmaceutical composition comprising an effective amount of a compound of formula I (as defined in claim 1), or a physiologically acceptable addition salt thereof, formulated with an aqueous solution of a physiologically acceptable bisulphite.

11. A process for the preparation of compounds as claimed in any one of claims 1 to 8 comprising bisulphite addition to a compound of formula I (as defined in claim 1).

12. A process as claimed in claim 11 comprising reacting said compound of formula I with a bisulphite ion providing substance in an aqueous medium, optionally in the presence of a phase transfer catalyst.

13. The use of compounds as claimed in any of claims 1 to 8 (wherein the or any counter cation is physiologically acceptable) for the manufacture of a therapeutic agent for use in the treatment of the human or non-human animal body to effect metaphase arrest of cell growth.

14. A process for the preparation of a pharmaceutical

composition comprising admixing a compound as claimed in any of claims 1 to 8 (wherein the or any counter cation is physiologically acceptable) with one or more pharmaceutical carriers or diluents.

15. Compounds of formula III

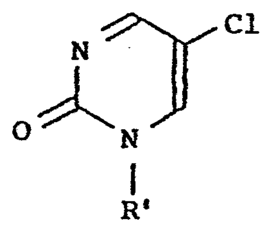

(III)

(wherein R' represents the group $-CH_2OR'^3$ (in which $R'^3$ represents a phenyl group substituted in the 4-position by a formyl group) or $-CH_2OCH_2R'^3$ in which $R'^3$ represents a phenyl group substituted in the 3- or 4- position by a methyl group or in the 3- and 4- positions by a chlorine atom)) and salts thereof, being compounds useful in the preparation of compounds as claimed in either of claims 1 and 2.